# EUROPEAN PATENT SPECIFICATION

(11) **EP 3 860 615 B1**
(45) Date of publication and mention of the grant of the patent: **18.09.2024**
(21) Application number: 19795336.7
(22) Date of filing: 07.10.2019
(51) Int. Cl.: A61K 31/704, A61K 36/73, A61P 3/04, A61K 36/77

(54) **ORAL COMPOSITION COMPRISING B-ESCIN AND THE USE THEREOF**
ORALE ZUSAMMENSETZUNG MIT B-ESCIN UND IHRE VERWENDUNG
COMPOSITION ORALE COMPRENANT DE LA B-ESCINE ET UTILISATION ASSOCIÉE

(30) Priority: 05.10.2018 PL 42731018
(43) Date of publication of application: 11.08.2021
(73) Proprietor: Escilab SP. Z O.O., 02-708 Warszawa (PL)
(72) Inventor: KOZIAK, Katarzyna, 02-708 Warszawa (PL)
(74) Representative: Patpol Kancelaria Patentowa Sp. z o.o.
(86) International application number: PCT/IB2019/058529
(87) International publication number: WO 2020/070732

(56) References cited:
- WO-A1-2018/096502
- PL-A1- 405 318
- HIDETO KIMURA ET AL: "Antiobese Effects of Novel Saponins from Edible Seeds of Japanese Horse Chestnut ( Aesculus turbinata BLUME) after Treatment with Wood Ashes", JOURNAL OF AGRICULTURAL AND FOOD CHEMISTRY, vol. 56, no. 12, 31 May 2008 (2008-05-31), US, pages 4783 - 4788, XP055654661, ISSN: 0021-8561, DOI: 10.1021/jf800340s
- JIANG-NING HU ET AL: "Anti-obesity Effects of Escins Extracted from the Seeds of <i>Aesculus turbinata</i> B<small>LUME</small> (Hippocastanaceae)", CHEMICAL AND PHARMACEUTICAL BULLETIN, vol. 56, no. 1, 1 January 2008 (2008-01-01), JP, pages 12 - 16, XP055654662, ISSN: 0009-2363, DOI: 10.1248/cpb.56.12
- GÜLCAN AVCI ET AL: "Effects of escin mixture from the seeds of Aesculus hippocastanum on obesity in mice fed a high fat diet", PHARMACEUTICAL BIOLOGY, vol. 48, no. 3, 17 March 2010 (2010-03-17), NL, pages 247 - 252, XP055654663, ISSN: 1388-0209, DOI: 10.3109/13880200903085466
- KIMURA H ET AL: "Identification of novel saponins from edible seeds of Japanese horse chestnut (Aesculus turbinata Blume) after treatment with wooden ashes and their nutraceutical activity", JOURNAL OF PHARMACEUTICAL AND BIOCHEMICAL ANALYSIS, ELSEVIER B.V, AMSTERDAM, NL, vol. 41, no. 5, 28 August 2006 (2006-08-28), pages 1657 - 1665, XP028005262, ISSN: 0731-7085, [retrieved on 20060828], DOI: 10.1016/J.JPBA.2006.02.031

## Description

### TECHNICAL FIELD

The present invention relates in general to a field of treatment and prevention of overweight and obesity. In particular the present invention is directed to an oral composition comprising β-escin (beta-escin) for use in prevention of extracellular to intracellular water ratio (ECW/ICW) increase in weight loss interventions in treatment of overweight and obesity. Therefore, the composition of invention has a beneficial effect on fluid distribution during weight loss. The composition of the invention not only improves ECW/ICW ratio but also induces anorectic effect and results in appetite suppression. Therefore, it is independently useful as overweight and obesity intervention.

### BACKGROUND ART

Obesity is a life-threatening disorder in which there is an increased risk of morbidity and mortality arising from concomitant diseases such as type II diabetes, hypertension, stroke, cancer and gallbladder disease.

The obesity pandemic is growing at an alarming rate with huge consequences for health and economy. In less than 35 years the worldwide prevalence of obesity has nearly doubled. In 2014, 11% of men and 15% of women in the world were obese as opposed to 5% for men and 8% for women in 1980. Today, more than 1.9 billion adults are overweight and of these, over 600 million are obese. Despite multiple educational and pharmacological approaches developed to treat obesity, none of the current treatments fully meets the patients' needs.

Eating disorders include conditions which may be characterized by abnormal eating habits. The abnormal eating habits may include either insufficient or excessive food intake to the detriment of physical and/or emotional health. Such eating disorders may include, for example, binge eating disorder, bulimia nervosa or anorexia nervosa. Although many eating disorders are associated with women, eating disorders are not gender specific.

Obesity has been defined in terms of body mass index ("BMI"). BMI is calculated as weight (kg)/[height (m)]². According to the guidelines of the U.S. Centers for Disease Control and Prevention ("CDC") and the World Health Organization ("WHO"), for adults over 20 years old, BMI falls into one of the following categories: below 18.5 kg/m² is considered underweight, 18.5-24.9 kg/m² is considered normal, 25.0-29.9 kg/m² is considered overweight, and 30.0 kg/m² and above is considered obese, see Table below (World Health Organization. Physical status: The use and interpretation of anthropometry. Geneva, Switzerland: World Health Organization 1995. WHO Technical Report Series).

| BMI | CLASSIFICATION |
|---|---|
| < 18.5 | Underweight |
| 18.5-24.9 | Normal |
| 25.0-29.9 | Overweight |
| 30.0-34.9 | Obesity (Class I) |
| 35.0-39.9 | Obesity (Class II) |
| >40 | Extreme Obesity (Class III) |

As the BMI increases there is an increased risk of death from a variety of causes that is independent of other risk factors. The most common diseases with obesity are cardiovascular disease (particularly hypertension), diabetes (obesity aggravates the development of diabetes), gall bladder disease (particularly cancer) and diseases of reproduction. Research has shown that even a modest reduction in body weight can correspond to a significant reduction in the risk of developing coronary heart disease.

There are problems however with the BMI definition in that it does not take into account the proportion of body mass that is muscle in relation to fat (adipose tissue). To account for this, obesity can also be defined on the basis of body fat content: greater than 25% in males and 30% in females. Obesity considerably increases the risk of developing cardiovascular diseases as well. Coronary insufficiency, atheromatous disease, and cardiac insufficiency are at the forefront of the cardiovascular complication induced by obesity. It is estimated that if the entire population had an ideal weight, the risk of coronary insufficiency would decrease by 25% and the risk of cardiac insufficiency and of cerebral vascular accidents by 35%. The incidence of coronary diseases is doubled in subjects less than 50 years of age who are 30% overweight. The diabetes patient faces a 30% reduced lifespan. After age 45, people with diabetes are about three times more likely than people without diabetes to have significant heart disease and up to five times more likely to have a stroke. These findings emphasize the inter- relations between risks factors for NIDDM and coronary heart disease and the potential value of an integrated approach to the prevention of these conditions based on the prevention of obesity (Perry, I. J., et al., BMJ 310, 560-564 (1995)).

Obesity is characterized not only by an increase in fat mass and, to a lesser extent, in fat-free mass, but also by an altered distribution of body fluid. Overweight and obese individuals have a greater volume of extracellular relative to intracellular fluid (ECF/ICF) compared to normal weight subjects. It is presently unknown why obesity is associated with a greater ECF/ICF ratio, nor whether the phenomenon represents a cause or consequence of obesity. Several mechanisms have been proposed to explain the body fluid perturbation observed in obesity. The phenomenon may reflect overhydration, excess total body water, related to disordered fluid regulation, oedema, hormones secreted by adipose tissue, a higher ECF/ICF ratio of adipose tissue, atrophied tissues or malnutrition (Disparate hydration of adipose and lean tissue requires a new model for body water distribution in man. Wang J, Pierson RN. J Nutr 1976; 106, 1687-1693; Increased extracellular water compartment, relative to intracellular water compartment, after weight reduction. Van Marken Lichtenbelt WD, Fogelholm M. J Appl Physiol 1999;87: 294-298; Relative overhydration of fat-free mass in post-obese vs never obese subjects. Leone PA, Gallagher D, Wang J, Heymsfield SB. Ann NY Acad Sci 2000; 904, 514-519. Alternatively, the excess extracellular volume might reflect cell dehydration, normal fluid shifts out of cells to the extracellular space in response to osmotic stress from plasma solute (Increased extracellular water compartment, relative to intracellular water compartment, after weight reduction. Van Marken Lichtenbelt WD, Fogelholm M. J Appl Physiol 1999;87: 294-298). It is well known that the extracellular volume is maintained at the expense of the intracellular space (Dehydration in the elderly: a short review. Lavizzo-Mourey RJ. J Natl Med Assoc 1987; 79, 1033-1038). Very important data on greater ECF/ICF ratio in obesity comes from the study of Stookey et al. (The altered fluid distribution in obesity may reflect plasma hypertonicity. Stookey, J. D., Barelay, D., Arieff, A., Popkin, B. M. European Journal of Clinical Nutrition 2007; 61, 190-199). The presented results suggest that the greater ECF/ICF ratio characteristic of obesity may reflect osmotic fluid shifts out of cells as opposed to overhydration. In the study overweight and obese individuals in this sample were significantly more likely to be hypertonic than their normal weight counterparts. The authors argue that over time, hypertonicity might promote weight gain and loss of lean mass (sarcopenic obesity) by decreasing lipolysis and energy expenditure. By increasing insulin resistance and oxidative stress, hypertonicity might promote metabolic disorders related to obesity, including diabetes and renal disease.

Importantly, in obese subjects after weight loss induced by dietary or surgical treatment the ECW/ICW ratio does not normalize, but it demonstrates a progressive increase.

For example in the study by Sergi et al. (Changes in Fluid Compartments and Body Composition in Obese Women after Weight Loss Induced by Gastric Banding. Ann Nutr Metab 2003;47:152-157) the ECW/ICW ratio changed from 0.78 at baseline to 0.90 after 6 months with a larger increase in the first weeks, when the weight loss was more rapid. In obese women treated with vertical banded gastroplasty, Mazariegos et al. (Body composition and surgical treatment of obesity. Effects of weight loss on fluid distribution. Ann Surg 1992;216:69-73) found an increased ECW/ICW ratio even though the increase was not significant. Van Marken Lichtenbelt and Fogelholm found an increase in the ECW/ICW ratio up to a year after the start of the 3-month weight-reduction program followed by 9 months of weight maintenance (Increased extracellular water compartment, relative to intracellular water compartment, after weight reduction. Van Marken Lichtenbelt WD, Fogelholm M. J Appl Physiol 1999;87: 294-298). The increased ECW/ICW ratio after weight reduction described by several surveys does not have a strong physiological basis. It is speculated that the relative expansion of extracellular water after weight loss could be due to obesity-related oedema, hormonal responses related to adipose tissue and an insulin-mediated sympathetic stimulation. It is also probable that obesity is accompanied by a primary and irreversible alteration in haemodynamics and fluid regulation persisting also after sustained weight loss.

Clinically significant weight loss is defined as weight loss of ≥5% of initial weight, which is an amount associated with improvements in weight related comorbidities, including diabetes, hypertension, osteoarthritis, and obstructive sleep apnea. Even in weight loss studies that are deemed successful, a proportion of individuals who participate either gain weight or do not achieve clinically significant weight loss. Studies of weight loss programs have shown a number of factors that are associated with clinically significant weight loss, including increased program participation, social support from friends, longer duration interventions, and older age (Factors Associated with Achievement of Clinically Significant Weight Loss by Women in a National Nonprofit Weight Loss Program. Mitchell, N. S. et al., Journal of Women's Health, 2017, 26, 911-917). An important issue associated with weight loss programmes is the problem of weight regain after weight loss (NIH working group report: Innovative research to improve maintenance of weight loss. MacLean P.S. et al., Obesity, 2015, 23, 7-15). Although many strategies have proven useful for inducing weight loss and reducing comorbidities, recidivism rates continue to be disturbingly high. Research by diverse groups of scientists over the last two decades have significantly improved our understanding of the physiology and behavioural mechanisms underlying the difficulty in weight loss and maintenance. Despite these advances, weight regain after weight loss remains the most substantial problem in obesity therapeutics.

One of the most promising strategies in obesity treatments is the reduction of energy intake through gastrointestinal mechanisms, which are regarded as possible targets for pharmacological or food-based approaches to weight management.

Clinical practice and experimental studies indicate that nutrient digestion and absorption inhibitors comprise an effective approach for weight loss (The role of lipid and carbohydrate digestive enzyme inhibitors in the management of obesity: a review of current and emerging therapeutic agents. Tucci S. et al., Diabetes, Metabolic Syndrome and Obesity: Targets and Therapy, 2010, 3, 125-143). Dietary fat is not directly absorbed by the intestine unless it has been subjected to the action of pancreatic lipase. Its activity is one of the most widely studied mechanisms for determining potential efficacy of anti-obesity agents. To date, only a few substances have been proven to interact directly with the lipases themselves, among them orlistat introduced to the market in 1999. The safety of orlistat has been proved in large clinical studies (Long-term inhibition of intestinal lipase by orlistat improves release of gut hormones increasing satiety in obese women. Olszanecka-Glinianowicz, M. et al., Pharmacol Rep, 2013, 65, 666-71; Efficacy of orlistat 60 mg on weight loss and body fat mass in USArmy soldiers. Smith, T.J. et al., J Acad Nutr Diet, 2012. 112, 533-40), however, its use is not free of undesirable effects (Macrocytic anemia and thrombocytopenia induced by orlistat. Palacios-Martinez, D. et al., Int J Endocrinol Metab, 2013, 11, e6721). For these reasons, there is an ongoing pursuit of new, safe and effective anti-obesity medications. Special attention is given to plant derived substances which provide a vast pool of pancreatic lipase inhibitors with clinical potential (Pancreatic lipase inhibitors from natural sources: unexplored potential. Birari, R.B. and Bhutani K.K., Drug Discov Today, 2007, 12, 879-89).

β-escin is a mixture of triterpene saponins isolated from the horse chestnut seeds (genus *Aesculus,* for example *Aesculus hippocastanum* L.). Although ethnopharmacological research provides evidence for its broad use to treat numerous diverse disorders, including bladder diseases, cough, diarrhea, dysmenorrhea and tinnitus (Aescin: pharmacology, pharmacokinetics and therapeutic profile. Sirtori CR. Pharmacol Res. 2001,44, 183-93), its current use is restricted mainly to venotonic and venoprotective indications (Horse-chestnut seed extract for chronic venous insufficiency. A criteria-based systematic review. Pittler MH and Ernst E. Arch Dermatol. 1998, 134, 1356-60; Horse chestnut seed extract for chronic venous insufficiency. Pittler MH and Ernst E. Cochrane Database Syst Rev. 2012;11:CD003230).

In 2006 Kimura et al. reported that β-escin block pancreatic lipase activity *in vitro* (*Identification of novel saponins from edible seeds of Japanese horse chestnut (Aesculus turbinata Blume)* after treatment with wooden ashes and their nutraceutical activity (Kimura et al., Journal of Pharmaceutical and Biomedical Analysis, 2006, 41, 1657-1665). Two years later the same group described anti-obesity effect of β-escin in mice model confirming that it is related to the inhibition of dietary fat digestion (Antiobese effects of novel saponins from edible seeds of Japanese horse chestnut (Aesculus turbinata BLUME) after treatment with wood ashes. Kimura, H. et al., J Agric Food Chem, 2008, 56, 4783-8). Similar observations were reported also by other researchers (Anti-obesity effects of escins extracted from the seeds of Aesculus turbinata BLUME (Hippocastanaceae). Hu, J.N. et al., Chem Pharm Bull (Tokyo), 2008. 56, 12-6; Effects of escin mixture from the seeds of Aesculus hippocastanum on obesity in mice fed a high fat diet. Avci, G. et al., Pharm Biol, 2010, 48, 247-52) who observed in animal studies that this plant-based drug significantly attenuated the elevation of the body weight, the mass of peritoneal adipose tissue and plasma triacylglycerol concentration, which was accompanied by higher contents of undigested fats in faces, indicating the effective inhibition of fat digestion.

### SUMMARY OF THE INVENTION

The invention is set out in the appended set of claims.

The present invention relates to an oral composition comprising an active ingredient and pharmaceutically acceptable excipients, wherein the active ingredient is β-escin, for use in the treatment of overweight and obesity.

In the preferred embodiment the composition further comprises chokeberry fruit extract.

According to the invention, the composition is for administration up to five times per day, preferably up to three times per day, most preferably once a day. In another embodiment the composition is for administration during or after a meal. Furthermore, in even more preferred embodiment the composition is for administration once per day during or after the first meal of a day or alternatively five times per day during or after any meal. Moreover, in one of the preferred embodiments the composition is in a solid form, preferably in a form of a tablet, capsule, powder or granules. In another preferred embodiment the composition is in a liquid form, preferably in a form of a solution, wherein, more preferable β-escin is present at a concentration between 20 to 200 mg/l, preferably between 50 mg/l to 75 mg/l, even more preferably at a concentration of 50 mg/l. In the preferred embodiment such β-escin is dissolved in the solvent selected from water or ethanol, preferably the solvent is water. The composition for use according to the present invention further comprises one or more additives selected from flavourings, colourings, preservatives, vitamins, sweeteners, antioxidants, and minerals. In some preferred embodiment the composition further comprises an anti-foaming agent and/or CO₂. In yet another preferred embodiment said composition constitutes the only weight loss intervention for treatment of overweight and obesity. According to the invention the daily dose of β-escin in the composition amounts from 2,5 mg to 8,5 mg, preferably amounts 4 mg to 6 mg, most preferably amounts to 5 mg.

The composition of the invention is advantageously used in prevention and treatment of overweight and obesity and coexisting aberrations of fluid distribution: 20 ml of composition of invention containing most preferably 1 mg of β-escin is administered as a single dose after meals up to five times daily for six to twelve weeks. Then, for the duration of the following twelve weeks the treatment is continued at the dosage of 20 ml of composition of invention containing preferably 1 mg of β-escin once per day after the main meal. After completing the six-month treatment, the composition of invention may be used only occasionally, after excessive meals, to prevent weight gain from sporadic overeating. In another advantages practical realisation, the composition of the invention is used in the solid form as for example capsule or a tablet. Daily dosage of β-escin in the composition administrated in solid form amounts from 2,5 mg to 8,5 mg, preferably amounts 4 mg to 6 mg, most preferably amounts to 5 mg.

### BRIEF DESCRIPTION OF DRAWINGS

The subject of the invention was illustrated in drawings.
Fig. 1 displays graphically the fat content in stool obtained from overweight subjects following the consumption of the composition of invention.
Fig. 2 displays graphically reduction of a fat content observed in obese subjects during consumption of the composition of the invention, wherein E is escin and AE is chokeberry juice concentrate with escin.
Fig. 3 displays graphically the TBW%, ECW%, ICW% and ECW/ICW ratio observed in overweight and obese subjects during consumption of the composition of the invention, wherein E is β-escin and AE is chokeberry juice concentrate with β-escin.

### DETAILED DESCRIPTION OF THE INVENTION

Compositions and methods of the invention are useful in the prevention and treatment of overweight and obesity by inducing appetite suppression leading to a potent and sustained decrease in food intake and body weight loss.

β-escin used in a composition of the present invention is a mixture of triterpene saponins isolated from horse chestnut seeds (genus *Aesculus,* for example *Aesculus hippocastanum,* L.). Although ethnopharmacological research provides evidence for its broad use to treat numerous diverse disorders, including bladder diseases, cough, diarrhea, dysmenorrhea and tinnitus, its current use is restricted mainly to venotonic and venoprotective indications. Despite the therapeutic significance of β-escin and the popularity of the drug, which in the United States and Europe remains one of the best-selling herbal extracts accounting for 226 million U.S. dollar-market in 2014 (IMS Kilochem), its exact mechanism of action remains to be fully elucidated.

The present inventor has observed and confirmed in trials that the composition according present invention has surprising effects in appetite suppression with concomitant weight loss, therefore it could be used in the prevention and treatment of overweight and obesity. Advantageously, the composition permits to reduce food intake due to its anorectic effect.

The highest dose of β-escin present in the composition according to the invention, when it is in a liquid form, amounts to 200 mg/l. However, the preferred concentration of β-escin in the liquid composition is 180 mg/l. Most preferably 50 mg/l of β-escin is provided to a subject in a in a single dose of 20 ml during or immediately after a meal, wherein the composition is administered up to five times a day, which amounts preferably to the dose of 5 mg of β-escin per day. The relative amount (dose) of β-escin in the composition is significant since this active ingredient is a potent inhibitor of pancreatic lipase. High dosing can result in steatorrhea or at the very least undesirable abdominal pain. It is therefore important that the dose of this compound is sufficient to achieve the desired therapeutic effect of suppressing appetite without inherently causing unwanted side effects. It is therefore very common that drugs for venous insufficiency containing high doses of escin (most commonly 20 mg in a form of tablet or capsule, taken 3 times a day to reach total daily dose of 120 - 240 mg) are indicated to be taken either two hours prior to a meal or three hours after a meal (https://www.doz.pl/leki/p1902-Aescin_tabletki).

First nutraceutical activities of escins were described by Kimura et al. who demonstrated their inhibitory action on pancreatic lipase and predicted anti-obese effects (Identification of novel saponins from edible seeds of Japanese horse chestnut (Aesculus turbinata Blume) after treatment with wooden ashes and their nutraceutical activity. Kimura et al. Journal of Pharmaceutical and Biomedical Analysis, 2006, 41, 1657-1665). Further data obtained from animal studies indicated that the anti-obesity effects were observed in mice fed with diets containing very high escins-containing saponin fraction (0,1% - 0,5%), i.e. a mouse of approximately 25-30 g in weight consumed daily 5 - 25 mg of saponins (Antiobese Effects of Novel Saponins from Edible Seeds of Japanese Horse Chestnut (Aesculus turbinata BLUME) after Treatment with Wood Ashes. Kimura et al., J. Agric. Food Chem. 2008, 56, 4783-4788). As clearly stated by the authors, the attenuating effect on body weight resulting from saponin treatment was not due to the reduced food intake but due to an inhibitory effect on lipase activity. Similar conclusions can be drawn from another animal study in which the authors used even higher doses of escins (up to 2% in diet in mice and up to 1g/kg body in rats): the change in body weight was not associated with decreased food consumption and was attributed to the inhibition of pancreatic lipase activity (Anti-obesity Effects of Escins Extracted from the Seeds of Aesculus turbinata BLUME (Hippocastanaceae). Hu et al., Chem. Pharm. Bull. 2008, 56, 12-16). Very high dose of escin, i.e. 100 mg/kg was administered to mice also in a study by Avci et al. (Effects of escin mixture from the seeds of Aesculus hippocastanum on obesity in mice fed a high fat diet. Avci et al. Pharmaceutical Biology, 2010, 48, 247-252). In concordance with the results of earlier studies, while the authors described the beneficiary effect of escin on the body weight in rats fed a high fat diet, they did not report any effect of the executed treatment on the food consumption. It should be underlined that in animal studies β-escin was administered in doses higher by three orders of magnitude as compared to the composition of invention.

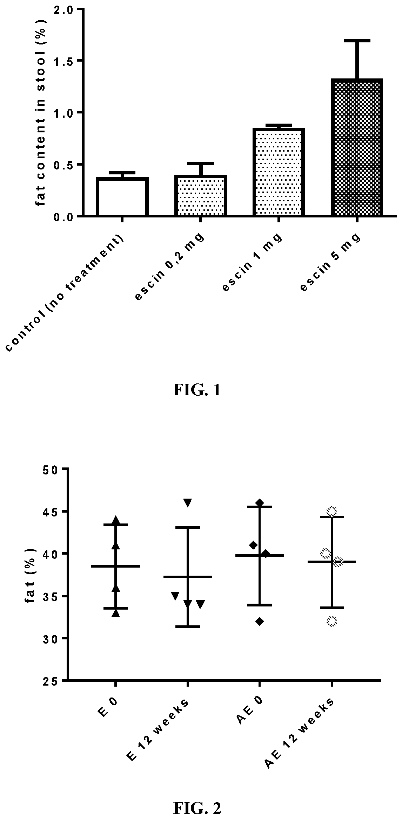

Very limited and not unequivocal human data linking escin treatment to appetite suppression comes mainly from patent documents. Polish patent application P.405318 discloses the use of composition comprising very low dose of β-escin (2 mg/l) and chokeberry fruit extract (200 g/l) to induce weight loss and describes diminished appetite in human subjects following the treatment. However, this publication does not provide sufficient information to clearly conclude that the observed weight loss and appetite inhibition is related to β-escin, and particularly β-escin provided in very low doses summing up to only 1 mg per day. As shown in Examples β-escin consumed by human subjects in a dose of 1 mg per day had no effect on lipid content in stool (Figure 1) and on satiety score. It is therefore more plausible that the effect described in P.405318 results from chokeberry component which has proven metabolic effects. Chokeberry extract is isolated from the fruits of *Aronia melanocarpa,* known i.e. for its antioxidant, hypocholesterolemic and hypoglycemic properties. Administration of chokeberry extract to diabetic rats attenuate hyperglycemia and hypertriglyceridemia (Hypoglycemic and hypolipidemic effects of Aronia melanocarpa fruit juice in streptozotocin-induced diabetic rats. Valcheva-Kuzmanova, S. et al., Methods Find Exp Clin Pharmacol, 2007, 29, 101-5). These effects, at least in part, are attributed to the natural phenolic constituents, which are beneficial in improving carbohydrate and lipid metabolism. Significant decrease in the absorption of nutrients as well as body weight gain was observed in animals fed a diet supplemented with chokeberry extract (Comparative Effect of Green Tea, Chokeberry and Honeysuckle Polyphenols on Nutrients and Mineral Absorption and Digestibility in Rats. Frejnagel S. and Wroblewska M., Ann Nutr Metab 2010;56:163-169). Even more important seem data showing that in rats fed with a fructose diet, which is a well-accepted animal model of obesity, extracts of *Aronia* improved blood glucose and serum lipid profile, reduced adipose tissue mass and body weight gain by regulating multiple genes in adipogenesis, glucose, lipid and inflammatory pathways. These included mRNA expression of CD36 - a fatty acid transporter, glycogen synthase and TNF-α (An extract of chokeberry attenuates weight gain and modulates insulin, adipogenic and inflammatory signalling pathways in epididymal adipose tissue of rats fed a fructose-rich diet. Qin B. and Anderson R.A. British Journal of Nutrition, 2012, 108, 581-587). In line with the above quoted articles remains patent application US 2010/0222422 A1 (T.J. Romero, B.Q., P.J. Miller, Dietary supplements containing extracts of Aronia and methods of using same to promote weight loss)*.* Furthermore, there are claims linking phytochemical constituents which comprise many edible berry fruits to increased satiety (Impact of bioflavonoids from berryfruits on biomarkers of metabolic syndrome. Lila M.A., Functional Foods in Health and Disease 2011, 1, 13-24).

In patent application US20080220101A1 β-escin is mentioned as one of components of compositions containing extracts of *Aloe vera* to induce satiety and/or reduce the appetite. Although β-escin does not appear in none of the claims of the patent document, it is mentioned in the detailed description of the invention as the active component of *Castanea sativa* - sweet chestnut and in the document wrongly named a horse chestnut. *Castanea sativa* is known for its antioxidant properties but it is not recognized as β-escin source, while it is the horse chestnut (genus *Aesculus*) that is a main known source of β-escin. Although due to the confusing description it is not clear if β-escin is included in the formulation, the purpose of including β-escin in the formulation is not revealed. Finally, the influence of plant extracts on feeding behaviour was tested in moth *Spodoptera littoralis* and not human subj ects.

Therefore, the strong appetite suppressing effect of the β-escin containing composition of the invention, observed in human subjects, should be considered as unexpected and could not be predicted from previously published studies.

The liquid composition of the invention comprising β-escin, at a concentration of less than 200 mg/l is disclosed for the first time in use as a medicament. The composition is particularly useful in the prevention or treatment of overweight and obesity resulting from appetite suppression and has beneficial effect on fluids distribution in overweight and obese subjects during weight loss.

The term "weight loss interventions" should be understood as any treatment method resulting in patient's weight loss, including but not limited to diet, surgical, pharmaceutical and psychological methods or combinations thereof. In view of the above, the term "treatment" should be understood as any process resulting in patient's weight loss, and which comprises any actions that leads to the required goal such as, for example, lifestyle management, pharmacotherapy and surgery. Further it can be understood as the use of an agent, procedure, or regimen, such as a diet, drug, surgery, or exercise, in an attempt to cure or mitigate a disease or condition associated with overweight and obesity.

The terms "overweight" and "obesity" are defined as abnormal or excessive fat accumulation that presents a health risk. A crude population measure of obesity is the body mass index (BMI), a person's weight (in kilograms) divided by the square of his or her height (in metres). A person with a BMI between 25-30 is considered overweight, and a person with a BMI of 30 or more is generally considered obese.

The term "chokeberry fruit extract" as used herein refers to any solid or liquid formulation obtained from chokeberry fruits. The liquid formulation might be, for example, a chokeberry fruit juice. In one preferred embodiment a chokeberry fruit extract is in a dried extract form. In other preferred embodiments, the composition of the invention comprises a chokeberry fruit juice concentrate. The chokeberry fruit extract, including fruit juice concentrate, may be provided in any form readily known to the skilled person, for example it may be in a dried extract form. Such juice concentrates are readily commercially available and characterised by a Brix value, wherein the extract typically comprises 65-68 _{°}Bx. Any suitable alternative supply sufficient to provide the same concentrate of chokeberry concentrate or extract is envisaged.

The following studies illustrate the most important findings related to the composition of invention.

### Example 1: In-vivo studies

### Satiation evaluation

Satiation is the process that brings a meal to an end. Verbal reports on the processes that bring a meal to an end indicate that 'fullness' and `boredom with taste' are two major reasons to stop eating. In the experiment, 30 healthy overweight adults were randomly assigned to three groups receiving: 1) composition of invention (1 mg β-escin in water); 2) composition comprising 0.2 mg β-escin in water; 3) placebo (sodium chloride). The subjects were instructed to take 20 ml of the provided composition after each main meal and two snacks, in total five times a day. As satiation is measured through the measurement of *ad libitum* food consumption, no food restrictions were advised. Throughout the 21-day, real-life study, at day 7, 14 and 21 the subjects were asked to complete five primary scales for self-reported appetite:
1) Hunger. The question: How hungry are you? The low and high anchor: "Not at all" and "Extremely", respectively;
2) Fullness. The question: How full are you? The low and high anchor: Not at all" and "Extremely", respectively;
3) Satiety. The question: How satiated are you? The low and high anchor: Not at all" and "Extremely", respectively;
4) Desire. The question: How strong is your desire to eat? The low and high anchor: "Very weak" and "Very strong", respectively;
5) Prospective consumption. The question: How much do you think you could eat right now? The low and high anchor: "Nothing at all" and "A very large amount", respectively.

The scales were linear (100 mm with 10 points where 1 was the low, and 10 the high anchor) and they were completed before and after the evening meal on the indicated days.

After the 21-day experiment and 7-day break, each group was assigned randomly for the new treatment, so at the end of the experiment each subject completed the scales for all the tested compositions.

The analysis of study results clearly indicated that only the group receiving the composition of invention reported significant hunger suppression, increased fullness and satiety, reduced desire to eat and declared strongly diminished prospective consumption (10 out of 10). In majority of cases (8 out of 10) the results of the treatment were observed on day 21, while the in two of the cases the results were observed on day 14 and on day 7, respectively. Importantly, all the subjects noted that the experienced changes led to reduced food consumption and therefore to reduced meal sizes. In the other groups only single cases of hunger suppression, increased fullness and satiety, reduced desire to eat and diminished prospective consumption were noted.

### Example 1A. Fat content in stool

To further investigate the unexpected satiety increasing and appetite decreasing effect of the β-escin, the inventor performed the measurement of lipid content in stool. It is well established that β-escin is an inhibitor of lipase activity and may lead to steatorrhea (fatty stools) in administered in high doses. In the performed tests, four obese patients were asked to ingest composition of invention (1 mg β-escin in 20 ml water); 0.2 mg β-escin in 20 ml water or 5 mg β-escin in 20 ml water, after each meal and two snacks, i.e. 5 times a day. The stool samples were collected on day 2. One-week break was ensured between the different treatments. As shown in the Figure, consumption of 5 mg and 25 mg of β-escin per day (i.e. five doses of 1 mg or 5 mg) led to considerable increase in the lipid content in stool.

However, the patients receiving the dose of 5 mg surprisingly did not notice any discomfort related to the increased lipid content in stool, whereas the patients receiving the highest dose of β-escin (25 mg per day) noticed symptoms such as loose stools, particularly after meals with high fat content, which was confirmed in the biochemical analyses.

### Example 1B. Weight loss

The group of 30 overweight (BMI 25,0 - 29,9) and obese (BMI >30) volunteers having body weight from 54 to 146 kg was recruited for a 3-month study (the initial treatment) aiming to assess the weight loss efficacy of the composition of invention (0,5-1,5 mg β-escin in water five times a day). Subsequently, in the next 3-month period 10 volunteers did not receive any treatment, 10 took the composition of invention only once a day after the biggest meal (2,5-8,5 mg β-escin per day in water) and 10 continued the initial treatment (0,5-1,5 mg β-escin in water five times a day). Importantly, at the time when the first dose of the composition of invention was administered, all the study subjects were consuming their normal diet. At the end of the first 3-month initial treatment: all of the 30 volunteers lost weight, of which 28 (93%) reached or surpassed the 5% value indicating a clinically significant weight loss. Further weight reduction was observed in both groups during the continuing treatment, although the values for the subjects continuing the initial treatment were higher as compared to the subjects receiving the composition of invention only once a day after the biggest meal (additional 5% vs 2% of initial body weight, mean values). In the group not receiving the treatment no weight regain was observed and in 4 subjects further slight weight loss was noted (1% of initial body weight). Moreover, in groups receiving the treatment reduced food consumption has been observed.

The results indicate that the composition of invention can be used as an efficient aid in weight management in overweight and obese subjects. Furthermore, no correlation between the BMI and weight loss efficacy was detected which indicates that β-escin content in the composition of invention (2,5-8,5 mg β-escin per day) is equally effective in overweight and obese patients and it is not necessary to scale up the dosage in adjustment to patient weight.

### Example 2

### Vitamin D levels

Due to a potential adverse effect of β-escin consumption on the absorption of fat soluble vitamins, related to β-escin induced reduction in lipid absorption, the inventor monitored the level of vitamin D (vitamin 25(OH)D) in randomly assigned six subjects treated with the composition of invention. During the duration of the tests vitamin D concentration in blood was assessed three times: at the beginning of the study prior to β-escin consumption, after 6 weeks and at the end of the study, i.e. after three months. The obtained results demonstrated that the treatment with the composition of invention had no effect on vitamin 25(OH)D concentration, which remained stable throughout the study in the range of ≥ 30-50 ng/ml (i.e. ≥ 75-125 nmol/l).

### Example 3

### Fat content reduction in obese subjects

Body composition was assessed by a Bioelectrical Impedance Analyser (BIA) BioScan (Maltron International Ltd) device. The manufacturer's in-build pre-programmed equations were used to measure body fat percentage (FAT%). All volunteers in the study were of BMI ≥30 (obese). As depicted in Fig. 2, 12-week treatment with the composition of invention (1 mg β-escin in water) with or without chokeberry juice concentrate (5 g) 3-5 times a day after meals had no clear effect on fat tissue reduction.

### Example 4

### Fluid distribution in overweight and obese subjects and its modifications during weight loss

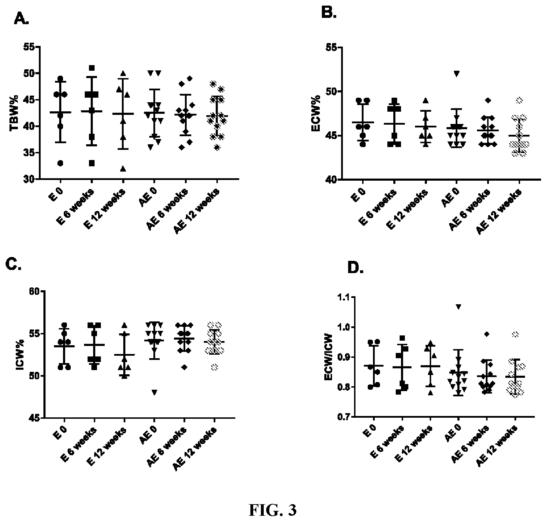

Body composition was assessed by a Bioelectrical Impedance Analyser (BIA) BioScan (Maltron International Ltd) device. The manufacturer's in-build pre-programmed equations were used to measure the percentage of total body water, as well as extracellular and intracellular water (TBW%, ECW% and ICW%). All volunteers in the study were of BMI ≥25 (overweight and obese) and they consumed the tested compositions 3-5 times a day after meals. As shown in Fig. 3A, the formulation of the composition, i.e. β-escin alone or with chokeberry juice concentrate had no effect on TBW%. However, surprisingly and unexpectedly there were differences between the evaluated groups in the ECW%, ICW% and in consequence in ECW/ ICW ratio. Formulation containing β-escin and chokeberry juice concentrate induced much more noticeable reduction in ECW% as compared to β-escin alone (Fig. 3 B), but had negligible effect on ICW%, whereas β-escin alone led to certain decrease in ICW% (Fig. 3 C). Consequently, the ECW/ICW ratio in the volunteers receiving composition containing 1 mg β-escin and 5 g chokeberry juice concentrate dropped, whereas in volunteers receiving composition of the present invention (1 mg β-escin in water) the ratio ECW/ICW remained unchanged. Changing aspects of fluid distribution parameters observed in both groups provide valid information about beneficial weight loss modifications obtained with β-escin, and particularly evident when used with chokeberry juice concentrate. According to current understanding of fluid distribution in obese subjects and its modifications during weight loss, a relative expansion of extracellular volume and higher ECW/ICW ratios do no normalize in individuals who lose weight, on the contrary, body fluid alterations progress. In the light of the above, β-escin induced appetite suppression leading to satisfactory weight loss and blocking, or as in case of β-escin with chokeberry juice concentrate - reversing fluid alterations represents an important invention in safe weight loss interventions.

The theoretical basis together with the in-vitro and human studies carried out by the inventor demonstrate a sound basis that the composition of the invention provides useful technical effects when administered to subjects, especially humans, but also animals. Due to the efficacy of the composition of the invention in weight management and no added sugars it may be advised as well for diabetic patients and elderly patients, as in prophylaxis against cardiovascular disease related to weight gain.

## Claims

1. Oral composition comprising an active ingredient and pharmaceutically acceptable excipients, wherein the active ingredient is β-escin, for use in treatment of overweight and obesity, wherein the composition is for administration up to five times per day, preferably up to three times per day, most preferably once a day, during or after a meal, and wherein daily dose of β-escin amounts from 2,5 mg to 8,5 mg.

2. The composition for use of claim 1, wherein it further comprises chokeberry fruit extract.

3. The composition for use of any of claims 1 or 2, wherein the composition is for administration once per day during or after the first meal of a day.

4. The composition for use of any of claims 1 or 2, wherein the composition is for administration five times per day during or after any meal.

5. The composition for use of any of claims 1 to 4, wherein the composition is in a solid form, preferably in a form of a tablet, capsule, powder or granules.

6. The composition for use according any of claims 1 to 4, wherein the composition is in a liquid form, preferably in a form of a solution.

7. The composition for use of claim 6, wherein β-escin is present at a concentration between 20 to 200 mg/l, preferably between 50 mg/l to 75 mg/l, even more preferably at a concentration of 50 mg/l.

8. The composition for use of claim 6 or 7, wherein β-escin is dissolved in the solvent selected from water or ethanol, preferably the solvent is water.

9. The composition for use of any of the previous claims further comprising one or more additives selected from flavourings, colourings, preservatives, vitamins, sweeteners, antioxidants, and minerals.

10. The composition for use of any of claim 1 to 4 and 6 to 9, wherein it further comprises an anti-foaming agent and/or CO₂.

11. The composition for use of any of the previous claims, wherein said composition constitutes the only weight loss intervention for treatment of overweight and obesity.

12. The composition for use of any of the previous claims, wherein daily dose of β-escin amounts from 4 mg to 6 mg, most preferably amounts to 5 mg.

## Patentansprüche

1. Oral zu verabreichende Zubereitung, die einen Wirkstoff und pharmazeutisch zulässige Hilfsstoffe enthält, wobei der Wirkstoff β-escin ist, zur Verwendung bei der Behandlung von Übergewicht und Fettleibigkeit,
wobei die Zubereitung bis zu fünfmal täglich, vorzugsweise bis zu dreimal täglich, im günstigsten Fall einmal täglich, während oder nach der Mahlzeit verzehrt wird, und
wobei die tägliche Dosis von β-Escin zwischen 2,5 mg und 8,5 mg beträgt.

2. Zubereitung zur Verwendung nach Anspruch 1, wobei sie außerdem Aroniafrucht-Extrakt enthält.

3. Zubereitung zur Verwendung nach einem der Ansprüche 1 oder 2, wobei die Zubereitung einmal täglich während oder nach der ersten Mahlzeit am Tag verzehrt wird.

4. Zubereitung zur Verwendung nach einem der Ansprüche 1 oder 2, wobei die Zubereitung fünfmal täglich während oder nach einer Mahlzeit verzehrt wird.

5. Zubereitung zur Verwendung nach einem der Ansprüche 1 bis 4, wobei die Zubereitung in fester Form vorliegt, vorzugsweise in Form von Tabletten, Kapseln, Pulver oder Granulat.

6. Zubereitung zur Verwendung nach einem der Ansprüche 1 bis 4, wobei die Zubereitung in flüssiger Form vorliegt, vorzugsweise in Form einer Lösung.

7. Zubereitung zur Verwendung nach Anspruch 6, wobei die Konzentration von β-escin zwischen 20 und 200 mg/l, vorzugsweise zwischen 50 mg/l und 75 mg/l, und weiter vorzugsweise 50 mg/l beträgt.

8. Zubereitung zur Verwendung nach Anspruch 6 oder 7, wobei β-escin in einem Lösungsmittel gelöst ist, das aus Wasser oder Ethanol ausgewählt ist, vorzugsweise das Lösungsmittel ist Wasser.

9. Zubereitung zur Verwendung nach einem der vorgenannten Ansprüche, die außerdem einen oder mehrere Zusatzstoffe enthält, ausgewählt aus Aromen, Farbstoffen, Konservierungsmitteln, Vitaminen, Süßstoffen, Antioxidantien und Mineralien.

10. Zubereitung zur Verwendung nach einem der Ansprüche 1 bis 4 und 6 bis 9, wobei sie außerdem ein Antischaummittel und/oder CO₂ enthält.

11. Zubereitung zur Verwendung nach einem der vorgenannten Ansprüche, wobei die vorgenannte Zubereitung die einzige Intervention zur Gewichtsabnahme für die Behandlung von Übergewicht und Fettleibigkeit darstellt.

12. Zubereitung zur Verwendung nach einem der vorgenannten Ansprüche, wobei die tägliche Dosis von β-Escin die Mengen von 4 mg bis 6 mg beträgt, im günstigsten Fall die Menge bis 5 mg.

## Revendications

1. Composition orale comprenant un ingrédient actif et des excipients pharmaceutiquement acceptables, dans laquelle l'ingrédient actif est la β-escine, pour le traitement du surpoids et de l'obésité,
dans laquelle la composition est destinée à être administrée jusqu'à cinq fois par jour, de préférence jusqu'à trois fois par jour, plus préférable une fois par jour, pendant ou après un repas, et
dans laquelle la dose journalière de β-escine est comprise entre 2,5 mg et 8,5 mg.

2. La composition de la revendication 1, dans laquelle elle comprend en outre de l'extrait de fruit d'aronia.

3. La composition à utiliser selon l'une des revendications 1 ou 2, dans laquelle la composition est destinée à être administrée une fois par jour pendant ou après le premier repas de la journée.

4. La composition à utiliser selon l'une des revendications 1 ou 2, dans laquelle la composition est destinée à être administrée cinq fois par jour pendant ou après un repas.

5. La composition à utiliser selon l'une des revendications 1 à 4, dans laquelle la composition se présente sous une forme solide, de préférence sous la forme d'un comprimé, d'une capsule, d'une poudre ou de granulés.

6. La composition à utiliser selon l'une des revendications 1 à 4, dans laquelle la composition est sous forme liquide, de préférence sous forme de solution.

7. Composition à utiliser selon la revendication 6, dans laquelle la β-escine est présente à une concentration comprise entre 20 et 200 mg/l, de préférence entre 50 mg/l et 75 mg/l, encore plus préférablement à une concentration de 50 mg/l.

8. Composition à utiliser selon la revendication 6 ou 7, dans laquelle la β-escine est dissoute dans le solvant choisi parmi l'eau ou l'éthanol, le solvant étant de préférence l'eau.

9. La composition à utiliser selon l'une des revendications précédentes comprend en outre un ou plusieurs additifs choisis parmi les arômes, les colorants, les conservateurs, les vitamines, les édulcorants, les antioxydants et les minéraux.

10. La composition à utiliser selon l'une des revendications 1 à 4 et 6 à 9, dans laquelle elle comprend en outre un agent antimousse et/ou du_{CO2}.

11. La composition à utiliser selon l'une des revendications précédentes, dans laquelle ladite composition constitue la seule intervention de perte de poids pour le traitement du surpoids et de l'obésité.

12. La composition à utiliser selon l'une des revendications précédentes, dans laquelle la dose quotidienne de β-escine est comprise entre 4 mg et 6 mg, plus préférable à 5 mg.
